Europäisches Patentamt

European Patent Office

Office européen des brevets

(19)

(11) **EP 1 442 702 A1**

(12) **EUROPÄISCHE PATENTANMELDUNG**

(43) Veröffentlichungstag:
**04.08.2004 Patentblatt 2004/32**

(51) Int Cl.⁷: **A61B 5/0295**

(21) Anmeldenummer: **04002376.4**

(22) Anmeldetag: **03.02.2004**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **03.02.2003 DE 10304338**

(71) Anmelder: **Soehnle-Waagen GmbH & Co. KG**
**71540 Murrhardt (DE)**

(72) Erfinder: **Nahm, Werner, Dr.**
**74426 Bühlerzell (DE)**

(54) **Vorrichtung zur plethysmographischen Bestimmung der Wiederauffüllzeit der Beinvenen**

(57)    Eine Vorrichtung dient zur Bestimmung der Lichtreflexion, insbesondere zur Bestimmung der Wiederauffüllzeit der Beinvenen. Eine verbesserte Vorrichtung dieser Art umfaßt einen Sensor (1) zum Erfassen der Lichtreflexion zu verschiedenen Zeitpunkten und eine Signalauswerteeinrichtung (10) zum Bestimmen des Zeitraums zwischen einem Ausgangszeitpunkt und einem Endzeitpunkt (Fig. 1).

Fig. 1

EP 1 442 702 A1

**Beschreibung**

Die Erfindung betrifft eine Vorrichtung zur Bestimmung der Lichtreflexion, insbesondere zur Bestimmung der Wiederauffüllzeit der Beinvenen. Sie betrifft ferner ein Verfahren und eine Vorrichtung zum Bestimmen eines Risikowerts, insbesondere eines Risikowerts für die Venenfunktion einer Person.

Aus der DE 199 01 149 A1 sind ein Verfahren und eine Vorrichtung zur nichtinvasiven Bestimmung von Blutvolumenänderungen, vorzugsweise in durchblutetem Gewebe, durch Messung der Lichtreflexion bzw. Lichttransmission bekannt. Bei dem Verfahren und der Vorrichtung werden ein Sensor mit einem Strahlungsempfänger und einer Strahlungsquelle, deren Strahlungsintensität einstellbar ist, verwendet. Photometrische Meßsysteme der aus der DE 199 01 149 A1 bekannten Art zur nichtinvasiven Bestimmung von Blutvolumenänderungen, die auch als Photoplethysmographen bzw. Licht-Reflexions-Rheographen bezeichnet werden können, werden in der medizinischen Diagnostik eingesetzt. Zur Bestimmung der Blutvolumenänderungen wird insbesondere infrarotes Licht verwendet, da in diesem Spektralbereich die Lichtabsorption des Blutes höher ist als diejenige des umliegenden Gewebes und sie nicht vom Grad der Sauerstoffsättigung des Blutes abhängig ist.

Die Bestimmung der Wiederauffüllzeit der Beinvenen mittels Licht-Reflexions-Rheographie (LRR) ist ein in der Venendiagnostik übliches Verfahren. Bei diesem Verfahren wird über eine optische Reflexionsmessung der Füllungsgrad der oberflächennahen Gefässe gemessen. Dies geschieht über die Messung der Lichtabsorption durch den roten Blutfarbstoff. Die Messung ist verbunden mit einer definierten Muskelkontraktion (Muskelpumpe), die das Blut aus den Beinvenen herauspreßt. Bei einer üblichen Durchführung des Verfahrens wippt der Patient zehnmal mit den Zehen oder den Fersen. In einer darauf folgenden Ruhephase, während der die durch die Muskelkontraktion leergepumpten Venen wieder aufgefüllt werden, kann das Reflexionssignal kontinuierlich gemessen und in Form einer Intensitäts-Zeit-Kurve dargestellt werden. Aus der Kurve kann der Arzt die Zeit bis zum vollständigen Wiederauffüllen der Venen bestimmen. Die so bestimmte Wiederauffüllzeit dient zur Einteilung des Meßergebnisses in definierte Insuffizienzstadien. Bislang ist die Durchführung, die Auswertung und die Interpretation der LRR-Messung nur durch speziell geschultes Personal möglich. Die gemessene Intensitäts-Zeit-Kurve muß von einem Arzt oder Apotheker mit speziellen Erfahrungen ausgewertet werden.

Aufgabe der Erfindung ist es, eine verbesserte Vorrichtung der eingangs angegebenen Art vorzuschlagen.

Erfindungsgemäß wird diese Aufgabe durch die kennzeichnenden Merkmale des Anspruchs 1 gelöst. Die Vorrichtung umfaßt einen Sensor zum Erfassen der Lichtreflexion zu verschiedenen Zeitpunkten und eine Signalauswerteeinrichtung zum Bestimmen des Zeitraums zwischen einem Ausgangszeitpunkt und einem Endzeitpunkt. Bei dem bestimmten Zeitraum handelt es sich insbesondere um die Wiederauffüllzeit der Venen bzw. Beinvenen.

Vorteilhafte Weiterbildungen sind in den Unteransprüchen beschrieben.

Vorzugsweise bestimmt die Signalauswerteeinrichtung als Endzeitpunkt den Zeitpunkt, zu dem die Lichtreflexion bzw. der Lichtreflexionswert bzw. die Intensität einen Anfangswert, den sie zu einem Anfangszeitpunkt aufweist, wieder erreicht oder annähernd wieder erreicht.

Nach einer weiteren vorteilhaften Weiterbildung bestimmt die Signalauswerteeinrichtung den Endzeitpunkt durch eine Auswertung von zu verschiedenen Zeitpunkten erfaßten Lichtreflexionswerten. Insbesondere kann der Endzeitpunkt durch eine Auswertung der Lichtrelexions-Zeit-Kurve bzw. Intensitäts-Zeit-Kurve bestimmt werden.

Die Signalauswerteeinrichtung kann zur Durchführung eines speziellen Auswerteverfahrens geeignet sein, welches den Endzeitpunkt bzw. den Endpunkt der Wiederauffüllzeit selbsttägig oder automatisch erkennt. Der Kurvenverlauf muß dazu nicht angezeigt werden, wenngleich dies nicht ausgeschlossen ist.

Eine Möglichkeit besteht darin, daß die Signalauswerteinrichtung einen Vergleich mit dem Anfangswert durchführt, insbesondere mit dem Anfangswert vor dem Beginn der beschriebenen Muskelkontraktion bzw. Pumpübung. Als Endzeitpunkt kann der Zeitpunkt definiert werden, zu dem der aktuelle Meßwert der Lichtreflexion bzw. der aktuelle Intensitätswert den Anfangswert erstmalig wieder erreicht oder zumindest annähernd wieder erreicht.

Es kann allerdings vorkommen, daß sich während der Messung Verschiebungen der optischen Meßstrecke ergeben oder daß sich die Gewebeperfusion durch physiologische Regelmechanismen verändert. Dies kann zur Folge haben, daß sich Signalveränderungen bezüglich der Anfangslage ergeben, die nicht ursächlich durch das Leerpumpen und Wiederauffüllen der Venen verursacht werden. Dadurch kann es vorkommen, daß der Anfangswert nicht mehr erreicht wird oder daß der Anfangswert bereits überschritten wird, bevor die eigentliche Wiederauffüllzeit abgeschlossen ist. Um diesen Problemen zu begegnen, kann die Signalauswerteeinrichtung ein Verfahren zum Bestimmen des Endzeitpunkts durchführen, das unabhängig vom Anfangspunkt zu Beginn der Messung erfolgt, insbesondere durch eine automatische Auswertung der Kurvenform, also der Lichtreflexions-Zeit-Kurve bzw. Intensitäts-Zeit-Kurve.

Eine weitere vorteilhafte Weiterbildung ist gekennzeichnet durch eine Signalauswerteeinrichtung zum Bestimmen eines Venenfunktionsindex aus dem bestimmten Zeitraum bzw. aus der Wiederauffüllzeit. Die Vorrichtung kann eine Anzeigeeinrichtung zum Anzeigen des Venenfunktionsindex umfassen.

Eine weitere vorteilhafte Weiterbildung ist dadurch gekennzeichnet, daß für einen bestimmten Zeitraum bzw. für eine Wiederauffüllzeit von bis zu einer Mindestzeit der Venenfunktionsindex einen Mindestwert beträgt. Die Mindestzeit beträgt vorzugsweise 1 bis 12 s (Sekunden), vorzugsweise 9 bis 11 s, vorzugsweise 10 s.

Eine weitere vorteilhafte Weiterbildung ist dadurch gekennzeichnet, daß für einen bestimmten Zeitraum bzw. eine Wiederauffüllzeit von über einer Höchstzeit der Venenfunktionsindex einen Höchstwert beträgt. Die Höchstzeit beträgt vorzugsweise 30 bis 40 s, vorzugsweise 32 bis 37 s, vorzugsweise 34,5 s.

Eine weitere vorteilhafte Weiterbildung ist dadurch gekennzeichnet, daß für bestimmte Zeiträume bzw. Wiederauffüllzeiten zwischen der Mindestzeit und der Höchstzeit der Venenfunktionsindex einen Zwischenwert beträgt. Vorzugsweise wird das Intervall zwischen der Mindestzeit und der Höchstzeit linear aufgeteilt. Es ist allerdings auch möglich, dieses Intervall in verschiedene Bereiche aufzuteilen und innerhalb dieser Bereiche jeweils eine lineare Beziehung zwischen dem bestimmten Zeitraum bzw. der Wiederauffüllzeit und dem Venenfunktionsindex zu realisieren. Der funktionale Zusammenhang wird dann durch einen Polygonzug realisiert. Hierdurch können insbesondere verschiedene Teilintervalle innerhalb des Intervalls verschieden stark bewertet werden. Eine weitere Möglichkeit besteht darin, die Zuordnung zwischen dem bestimmten Zeitraum bzw. der Wiederauffüllzeit und dem Venenfunktionsindex durch eine kurvenförmige Funktion zu realisieren.

Der Mindestwert kann auf 10 und der Höchstwert auf 100 festgesetzt werden. Hierdurch ergibt sich eine prozentuale Einteilung.

Wenn das Intervall zwischen der Mindestzeit und der Höchstzeit linear aufgeteilt wird, bestimmt die Signalauswerteeinrichtung den Venenfunktionsindex V vorzugsweise nach der Formel

$$V = a \cdot (T - b).$$

Hierin bedeuten:

V = Venenfunktionsindex
a = Multiplikationsfaktor
T = bestimmter Zeitraum bzw. Wiederauffüllzeit
b = Verschiebungswert.

Vorteilhaft ist es, die Werte a = 11/3 und b = 7,25 einzusetzen. Daraus ergibt sich folgende Formel:

$$V = 11/3 \, (T - 7{,}25)$$

Daraus ergibt sich für T = 10 ein Venenfunktionsindex V = 10. Für T < 10 wird V = 10 festgesetzt. Für T = 34,5 s ergibt sich V = 100. Für T > 34,5 s wird V = 100 festgesetzt. Dadurch wird der Venenfunktionsindex auf das Intervall zwischen 10 und 100 begrenzt: $10 \leq V \leq 100$.

Die Formelgrößen a (Multiplikationsfaktor) und b (Verschiebungswert) können von dem bestimmten Zeitraum bzw. der Wiederauffüllzeit T abhängig sein. Auf diese Weise kann eine Zuordnung zwischen dem bestimmten Zeitraum bzw. der Wiederauffüllzeit T und dem Venenfunktionsindex V realisiert werden, die nicht einer linearen Aufteilung entspricht, sondern einem Polygonzug oder einem kurvenförmigen Funktionszusammenhang.

Nach einer weiteren vorteilhaften Weiterbildung umfaßt die Vorrichtung eine Signalauswerteeinrichtung zum Bestimmen eines Anzeigewerts aus dem bestimmten Zeitraum bzw. der Wiederauffüllzeit oder aus dem Venenfunktionsindex und eine Anzeigeeinrichtung zum Anzeigen dieses Anzeigewerts.

Vorteilhaft ist es, wenn der Anzeigewert mehrere Bewertungsgrößen umfaßt. Vorzugsweise umfaßt der Anzeigewert drei Bewertungsgrößen. Dabei handelt es sich vorzugsweise um die Werte "gut", "ausreichend" und "mangelhaft". Diesen Werten können die Farben rot (mangelhaft), gelb (ausreichend) und grün (gut), in Analogie zu den Ampelfarben, zugeordnet werden.

Vorzugsweise weist der Anzeigewert den Bewertungsgröße "mangelhaft" für bestimmte Zeiträume bzw. Wiederauffüllzeiten bis zu 25 s auf, den Wert "ausreichend" für bestimmte Zeiträume bzw. Wiederauffüllzeiten von 25 s bis 31,5 s und den Wert "gut" für bestimmte Zeiträume bzw. Wiederauffüllzeiten von über 31,5 s. Anstelle der genannten Grenzen von 25 s und 31,5 s können geringfügig abweichende Werte verwendet werden.

In den Richtlinien zur Licht-Reflexions-Rheographie (LRR) der Deutschen Gesellschaft für Phlebologie wird folgende Einteilung der Auffüllzeit vorgenommen:

| | |
|---|---|
| Venöse Insuffizienz Klasse III | < 10 s. |
| Venöse Insuffizienz Klasse II | 10 - 20 s. |
| Venöse Insuffizienz Klasse I | < 20 - 25 s. |
| Gesund | > 25 s. |

Diese Einteilung trifft zwar für die Mittelwerte einer größeren Population zu, die konkreten individuellen Werte schwanken jedoch stark, so daß aus einer einzelnen Messung keine Einteilung in einen Schweregrad vorgenommen werden kann.

Die Ursachen für die Schwankungsbreite der gemessenen Auffüllzeiten sind in den physiologischen Änderungen des Venensystems bzw. in diversen physiologischen Regelvorgängen begründet. Die Messung der Auffüllzeit selbst erfolgt technisch mit einer gegenüber den physiologischen Änderungen höheren Genauigkeit von mindestens einer Größenordnung (Zehnerpotenz). Eine Einzelmessung ist somit lediglich als Ausdruck des momentanen physiologischen Status des Venensystems bzw. dessen momentanen Grades der Funktionstüchtigkeit anzusehen.

Über die Größe und Variabilität der Auffüllzeit können folgende Aussagen getroffen werden:

- Bei schweren Insuffizienzen mit Auffüllzeiten < 10 s ist die Variabilität der Auffüllzeiten sehr gering. Das Venensystem ist bereits derart geschädigt, daß in ihm keine Regulationsvorgänge mehr stattfinden können (überdehnte und verhärtete Venenwände) oder daß sich Regelvorgänge kaum hämodynamisch auswirken (da die Venen zu großkalibrig sind). In derartigen Fällen ist deshalb die Ermittlung eines sich ändernden Venenstatus nicht von Vorteil.

- Bei Personen mit mittlerer und leichter Veneninsuffizienz findet man in der Regel eine große Variabilität der Venenfunktion und somit der Auffüllzeit. Eine Person kann beispielsweise am Morgen mit regeneriertem Venensystem eine uneingeschränkte Venenfunktion aufweisen, während am Abend eine deutliche Veneninsuffizienz feststellbar ist. In diesen Fällen ist die Ermittlung der momentanen Venenfunktion sehr sinnvoll.

- Bei leichten Veneninsuffizienzen findet man häufig auch Auffüllzeiten, die nur wenig oberhalb 25 s liegen. Diese sind zwar formal (entsprechend den medizinischen Richtlinien) als nicht krankhaft einzuordnen, deuten sehr oft bereits auf eine entstehende Insuffizienz hin.

- Venengesunde haben in der Regel sehr lange Auffüllzeiten, meist > 35 s. Hier schließen die Venenklappen, und die Auffüllzeit wird nur durch die allerdings sehr variable arterielle Durchblutung bestimmt. Auffüllzeiten > 35 s und deren Änderungen sind deshalb für die Darstellung der Venenfunktion nicht von Interesse.

Aus den dargelegten Gründen ist deshalb die Einteilung der Auffüllzeit in hart abgegrenzte zeitliche Intervalle nicht sinnvoll. Zur quantitativen Bewertung der Venenfunktion wird gemäß der beschriebenen vorteilhaften Weiterbildung der Erfindung der numerische Wert der Auffüllzeit im Intervall von 10 s bis etwa 35 s betrachtet werden, wobei dieses Intervall geringfügig abgeändert werden kann.

Der beschriebene Venenfunktionsindex ermöglicht eine verbesserte Beurteilung. Durch ihn wird die Anschaulichkeit der Ergebnisse erhöht. Es werden nur diejenigen Intervalle dargestellt, die die Venenfunktion charakterisieren. Der Venenfunktionsindex leitet sich direkt aus der Auffüllzeit ab und hat folgende Eigenschaft:

(1) Bei einer Auffüllzeit von 10 s oder weniger (Grenze zur Insuffizienz Klasse III) wird der Venenfunktionsindex auf einen Mindestwert festgesetzt, beispielsweise auf den Mindestwert 10 oder 10%. Ein kleinerer Index sollte nicht ausgegeben werden.

(2) Eine Auffüllzeit von 25 s liegt an der Grenze zum Normal befund. Für diese Auffüllzeit oder eine geringfügig davon abweichende Auffüllzeit kann ein Venenfunktionsindex festgelegt werden, der einem ersten Zwischenwert entspricht, beispielsweise ein Venenfunktionsindex von 65 oder 65%. Werte des Venenfunktionsindex, die kleiner als dieser Zwischenwert sind, werden als "mangelhaft" eingestuft.

(3) Für Auffüllzeiten bis zu einem weiteren Zwischenwert von vorzugsweise 31,5 s oder einem geringfügig davon abweichenden Wert wird der Venenfunktionsindex auf einen zweiten, erhöhten Zwischenwert festgesetzt, beispielsweise auf 90 oder 90%. Zwischen dem ersten Zwischenwert und dem zweiten, erhöhten Zwischenwert liegt ein Unsicherheitsbereich, in dem die Wahrscheinlichkeit einer Veneninsuffizienz hoch ist. Allerdings kann auch eine erhöhte Hautdurchblutung zu einem Venenfunktionsindex in diesem Bereich führen. Deshalb wird dieser Bereich als "ausreichend" eingestuft.

(4) Für Wiederauffüllzeiten über einem Höchstwert von beispielsweise 34,5 s oder einem geringfügig davon abweichenden Wert wird der Venenfunktionsindex auf den Höchstwert festgesetzt, beispielsweis auf 100 oder 100%. Für Werte des Venenfunktionsindex über dem zweiten, erhöhten Zwischenwert und erst recht über dem Höchstwert kann davon ausgegangen werden, daß die Venenfunktion nicht gestört ist. Dieser Bereich wird als "gut" eingestuft.

Bei der Verwendung der genannten Grenzen oder hiervon geringfügig abweichender Grenzen entspricht der Wert "mangelhaft" einer Insuffizienz der Klassen I, II und III. Die Werte "ausreichend" und "gut" entsprechen dem Richtlinienwert "gesund" (> 25 s). Innerhalb des Richtlinienwerts "gesund" wird allerdings in der erläuterten Weise differenziert.

Der Anzeigewert kann auch aus dem Venenfunktionsindex bestimmt werden. Der Venenfunktionsindex führt zu einer leichten Erkennung interessanter Bereiche. Dennoch kann es für den ungeschulten Benutzer schwierig sein, das momentane Meßergebnis bzw. den ermittelten Venenfunktionsindex einem medizinisch relevanten Risikobereich zuzuordnen.

Hiervon ausgehend kann der Anzeigewert, vorzugsweise als Ampelwert in einer der drei Farben grün, gelb oder rot, anstelle des Venenfunktionsindex oder auch zusätzlich zu diesem angezeigt werden. Besonders vorteilhaft ist es, wenn der Anzeigewert durch Darstellung einer der leicht erfassbaren Ampelfarben grün, gelb oder rot angezeigt wird. Die Zuordnung zu einem Anzeigewert, insbesondere zu einer der Farben grün, gelb oder rot, kann durch eine spezielle Gestaltung eines Displays oder von Teilen eines Displays mit nahe bei diesem Display angebrachten Markierungen, insbesondere Farbmarkierungen, oder durch Ansteuerung aktiver, insbesondere farbiger Leuchtelemente erfolgen. Die formale Zuordnung eines bestimmten Zeitraums bzw. einer Wiederauffüllzeit zu einem Anzeigewert, insbesondere zu einer Ampelfarbe, erfolgt analog zum Venenfunktionsindex durch eine Kombination des bestimmten Zeitraums bzw. des Venenfunktionsindex mit den Insuffizienzklassen. Um eine feinere Unterteilung zu erreichen, können die Wertebereiche des Anzeigewerts, insbesondere die drei Ampelbereiche, graphisch noch in Unterelemente und/oder Unterbereiche aufgegliedert sein.

Eine weitere vorteilhafte Weiterbildung ist gekennzeichnet durch ein Betätigungselement zum Aktivieren und/oder Steuern der Vorrichtung. Bei dem Betätigungselement kann es sich insbesondere um einen Betätigungsknopf oder eine Taste handeln, insbesondere eine Drucktaste.

Vorteilhaft ist es, wenn nur ein einziges Betätigungselement zum Aktivieren und Steuern der Vorrichtung vorhanden ist. Hierdurch kann die Bedienung vereinfacht werden. Insbesondere kann eine Einhandbedienung ermöglicht werden. Ferner kann die Sicherheit der Bedienung erhöht werden.

Eine weitere vorteilhafte Weiterbildung ist dadurch gekennzeichnet, daß der Sensor einen Sender, insbesondere eine Strahlungsquelle, und einen Empfänger, insbesondere einen Strahlungsempfänger, umfaßt. Bei der Strahlung handelt es sich insbesondere um Lichtstrahlung, vorzugsweise um Infrarotstrahlung. Vorzugsweise umfaßt der Sensor nur einen Sender und nur einen Empfänger.

Der Sensor kann einen Potentialkontakt umfassen. Hierdurch kann ein elektrischer Kontakt zu der Person hergestellt werden. Der Potentialkontakt kann durch einen Ohmschen Kontakt realisiert werden, insbesondere durch einen Kontaktstift. Der Kontaktstift ist vorzugsweise aus Messing. Es ist ferner möglich, den Kontaktstift derart auszugestalten, daß er gleichzeitig zur Befestigung dient, beispielsweise zur Befestigung einer Leiterplatte oder Platine an einem Teil der Vorrichtung oder des Sensors, beispielsweise an einem Deckel des Sensors.

Vorteilhaft ist es, wenn der Sensor eine Kontaktfläche umfaßt. Die Kontaktfläche ist vorzugsweise ringförmig. Es sind allerdings auch andere Formen für die Kontaktfläche möglich. An der vorzugsweise ringförmigen Kontaktfläche kann ein Klebering vorgesehen sein. Dabei kann es sich um ein folienartiges Teil, vorzugsweise aus Kunststoff, handeln, das auf beiden Seiten eine Klebeschicht aufweist. Der Sensor kann auf diese Weise durch den beidseitig klebenden Klebering auf einfache Weise an der Person befestigt werden.

Vorzugsweise ist der Sensor und/oder die zu untersuchende Oberfläche bzw. Haut gekühlt. Die Oberflächentemperatur der zu untersuchenden Oberfläche bzw. der Haut wird durch die Wärmeproduktion des Körpers und durch die Wärmeabgabe über die Hautoberfläche bestimmt. Ein Abdecken eines bestimmten Oberflächenareals bzw. Hautareals durch den Sensor kann die Wärmeabstrahlung beeinträchtigen oder verhindern und dadurch zu einer Temperaturerhöhung unter dem Sensor führen. Hierdurch kann eine Mehrdurchblutung angeregt werden, die über den Sensor erfaßt werden kann, was wiederum zur Folge haben kann, daß eine zusätzliche Blutvolumenänderung gemessen wird, die die Wiederauffüllkurve verfälscht. Diese Gefahr kann dadurch erhöht werden, daß es im allgemeinen wünschenswert oder erforderlich ist, die Strahlungsquelle möglichst nahe an die zu untersuchende Oberfläche bzw. Haut der Person heranzubringen. Es ist daher vorteilhaft, den Sensor und/oder die zu untersuchende Oberfläche bzw. Haut zu kühlen bzw. den Sensor derart auszugestalten, daß eine Kühlung des Sensors und/oder der zu untersuchenden Oberfläche bzw. Haut ermöglicht wird. Dadurch kann der beschriebene Isolationseffekt verhindert werden.

Vorteilhaft ist es, wenn der Sensor belüftbar ist. Um dies zu erreichen, können in und/oder an dem Sensor Belüftungskanäle vorgesehen sein. Insbesondere können in der vorzugsweise ringförmigen Kontaktfläche Kerben vorgesehen sein. Die Kerben sind vorzugsweise derart ausgebildet, daß Luftkanäle entstehen. Hierdurch kann der Sensor belüftet und damit gekühlt werden. Stattdessen oder zusätzlich können durch den Sensor und/oder das Sensorgehäuse hindurchführende Belüftungskanäle vorhanden sein.

Eine weitere vorteilhafte Weiterbildung ist dadurch gekennzeichnet, daß der Sensor, insbesondere die vorzugsweise ringförmige Kontaktfläche des Sensors ein wärmeleitendes Material umfaßt. Dabei handelt es sich vorzugsweise um ein Metall oder ein sonstiges Material mit einer hohen Wärmeleitfähigkeit. Durch die Ableitung der Wärme kann der Sensor gekühlt werden. Wenn als wärmeableitendes Material ein Metall verwendet wird, kann der weitere Vorteil erreicht werden, daß ein gesonderter Kontaktstift entbehrlich ist.

Die Erfindung betrifft ferner ein Verfahren zum Bestimmen eines Risikowerts, insbesondere eines Risikowerts für die Venenfunktion einer Person, und eine Vorrichtung zur Durchführung eines derartigen Verfahrens.

Die apparative Venenfunktionsdiagnostik durch Messung der Wiederauffüllzeit mittels plethysmographischer Messmethoden, z. B. der Lichtreflexionsrheographie, beschränkt sich auf die Bestimmung des Momentanzustandes zum Zeitpunkt der Messung. Die gemessene Wiederauffüllzeit hängt jedoch neben der Funktion der Venenklappen auch noch von weiteren hämodynamischen Bedingungen wie z. B. dem arteriellen Zufluss oder der subcutanen Perfusion ab. Weiterhin unterliegt die Venenfunktion selbst Schwankungen, die physiologischer oder pathologischer Natur sein können bzw. die aufgrund äußerer Einflüsse wie z. B. belastender oder entlastender Tätigkeiten entstehen.

Aus diesem Grund kann durch eine Momentanmessung nur unzureichend auf das Vorliegen einer Venenfunktionsschwäche geschlossen werden. Die Situation ist vergleichbar mit der Blutdruckmessung, bei der über eine einmalige Messung nicht automatisch auf einen Bluthochdruck geschlossen werden kann.

Andererseits liegt gerade in der Veränderung der Wiederauffüllzeit in Abhängigkeit von Belastungs- oder Entlastungssituationen oder in tageszeitlichen Schwankungen eine wichtige Information darüber, wie hoch das Risiko für eine Venenfunktionsschwäche ist, und zwar schon im Vorfeld einer klinisch manifestierten Veneninsuffizienz.

Hiervon ausgehend besteht eine weitere Aufgabe der Erfindung darin, ein Verfahren und eine Vorrichtung zum Bestimmen eines Risikowerts, insbesondere eines Risikowerts für die Venenfunktion einer Person vorzuschlagen, die eine bessere Risikobewertung für das Vorliegen einer Venenfunktionsschwäche ermöglichen.

Erfindungsgemäß wird diese Aufgabe bei einem Verfahren der genannten Art dadurch gelöst, daß die Wiederauffüllzeit der Beinvenen einer Person zu verschiedenen Zeiten bestimmt wird, daß die bestimmten Wiederauffüllzeiten und/oder aus der jeweiligen Wiederauffüllzeit bestimmte Venenfunktionsindexwerte gespeichert werden und daß aus den Wiederauffüllzeiten bzw. Venenfunktionsindexwerten ein Risikowert bestimmt wird. Eine erfindungsgemäße Vorrichtung der genannten Art umfaßt eine Vorrichtung zum Bestimmen der Wiederauffüllzeit, insbesondere der Wiederauffüllzeit der Beinvenen, einen Speicher zum Speichern der zu verschiedenen Zeiten bestimmten Wiederauffüllzeiten und/oder zum Speichern eines aus der jeweiligen Wiederauffüllzeit bestimmten Venenfunktionsindex und eine Risikowert-Bestimmungseinrichtung zum Bestimmen des Risikowerts aus den Wiederauffüllzeiten bzw. den Venenfunktionsindexwerten.

Bei der Vorrichtung zum Bestimmen der Wiederauffüllzeit handelt es sich insbesondere um eine erfindungsgemäße Vorrichtung zur Bestimmung der Lichtreflexion, insbesondere zur Bestimmung der Wiederauffüllzeit der Beinvenen, die durch einen Sensor zum Erfassen der Lichtreflexion zu verschiedenen Zeitpunkten und eine Signalauswerteinrichtung zum Bestimmen des Zeitraums zwischen einem Ausgangszeitpunkt und einem Endzeitpunkt gekennzeichnet ist. Es ist allerdings auch möglich, eine andere Vorrichtung oder ein anderes Verfahren zum Bestimmen der Wiederauffüllzeit, insbesondere der Wiederauffüllzeit der Beinvenen, zu verwenden.

Um die Aussagekraft von plethysmographischen Messungen zur Venenfunktionsdiagnostik zu verbessern und eine Risikoeinschätzung für das Vorliegen einer Venenfunktionsschwäche zu ermöglichen, wird erfindungsgemäß vorgeschlagen; Messungen zu verschiedenen Zeitpunkten durchzuführen. Dazu wird ein Messsystem vorgeschlagen, welches für eine Langzeitüberwachung geeignet ist.

Bei dem erfindungsgemäßen Verfahren werden vorzugsweise die Zeiten, zu denen die Wiederauffüllzeiten bestimmt werden, gespeichert.

Der Risikowert kann durch ein Auswerteverfahren bestimmt werden. Vorzugsweise handelt es sich um ein statistisches Auswerteverfahren.

Eine weitere vorteilhafte Weiterbildung ist dadurch gekennzeichnet, daß der Risikowert mehrere Bewertungsgrößen umfaßt, vorzugsweise drei Bewertungsgrößen, vorzugsweise die Bewertungsgrößen "gut", "gefährdet" und "hohes Risiko".

Die erfindungsgemäße Vorrichtung ist vorzugsweise dadurch gekennzeichnet, daß die Zeiten, zu denen Wiederauffüllzeiten bestimmt werden, in dem Speicher gespeichert werden.

Eine weitere vorteilhafte Weiterbildung ist dadurch gekennzeichnet, daß der Risikowert mehrere Bewertungsgrößen umfaßt, vorzugsweise drei Bewertungsgrößen, vorzugsweise die Bewertungsgrößen "gut", "gefährdet" und "hohes Risiko".

Ein geeignetes Auswerteverfahren zum Bestimmen des Risikowerts ermöglicht eine statistische Auswertung der Messdaten. Es liefert einen Risikowert. Die Risikoeinschätzung anhand des Risikowerts sollte von einem medizinisch nicht geschulten Leien in einer einfachen Selbstandwendung durchführbar sein.

Die erfindungsgemäße Vorrichtung kann ein Messsystem umfassen, bestehend aus

    1. einem Meßgerät zum Bestimmen der Wiederauffüllzeit, beispielsweise einem Licht-Reflexions-Rheographie-Gerät, vorzugsweise zum mobilen Einsatz, welches vorzugsweise automatisch arbeitet und eine Größe ermittelt, die ein Maß für die aktuelle Venenfunktion darstellt, beispielsweise die Wiederauffüllzeit oder eine davon abgeleitete Größe, beispielsweise einen, bzw. den erläuterten Venenfunktionsindex, und

2 einer Möglichkeit zur Speicherung der Meßwerte bzw. der abgeleiteten Werte in Verbindung mit einer Zeitinformation, also einer Information über den Zeitpunkt, zu dem der jeweilige Meßwert bestimmt worden ist. Eine Möglichkeit besteht darin, ein Meßprotokoll zu erstellen, in welches die Meßwerte ähnlich wie bei einer Fieberkurve eingetragen werden, oder einen analogen oder digitalen Speicherbaustein oder eine spezielle Software auf einem Computer, insbesondere einem üblichen PC. Die Übertragung der Daten kann manuell, über Datenleitungen oder auch mittels drahtloser Datenübertragung erfolgen.

Das Auswerteverfahren kann aus folgendem bestehen:

1. Die Bestimmung statistischer Kenngrößen aus den gespeicherten Daten. Diese Kenngrößen können beispielsweise den Mittelwert und die Varianz der Venenfunktion über einen bestimmten Zeitraum beschreiben. Die Bestimmung einer statistischen Kenngröße kann auch dadurch erfolgen, daß abgezählt wird, wie viele der Meßwerte aus einem bestimmten Zeitintervall in vorgegebenen Intervallbereichen der Wiederauffüllzeiten und/oder des Venenfunktionsindex liegen. Beispielsweise kann die Wiederauffüllzeit in mehrere Bereiche eingeteilt werden oder eine abgeleitete Größe wie ein oder der beschriebene Venenfunktionsindex können in mehrere Venenfunktionskategorien eingeteilt werden.

2. Die Bestimmung eines Risikowertes aus den statistischen Kenngrößen. Dies kann über eine Einteilung in verschiedene Risikogruppen erfolgen, beispielsweise in drei Gruppen "gut (normal)", "gefährdet" und "hohes Risiko (insuffizient)". Beispielsweise kann die Einstufung "gut (normal)" erfolgen, wenn die Wiederauffüllzeit ausschließlich oder überwiegend im gesunden Bereich ist, vorzugsweise größer als 32s ist. Die Einstufung "gefährdet" kann beispielsweise erfolgen, wenn die Venenfunktion belastungsabhängige Schwankungen aufweist und häufig in einen Grenzbereich absinkt, beispielsweise in einem Grenzbereich, der bei Wiederauffüllzeiten zwischen 25 und 31,5 s liegt, bzw. zeitweise sogar in dem Bereich der DGP-Insuffizienz-Klassen I, II und III bei unter 25 s absinkt. Die Einstufung "hohes Risiko (insuffizient)" kann beispielsweise erfolgen, wenn die Venenfunktion auch in Entlastungssituationen permanent oder überwiegend im Bereich der DGP-Insuffiezienz-Klassen I, II und III bei unter 25 s liegt.

Das Verfahren, daß in der Vorrichtung verkörpert sein kann, kann durch 42 Messungen je Bein über einen Zeitraum von 14 Tagen erfolgen, z.B. über drei Messungen pro Tag und pro Bein über eine Zeitraum von 14 Tagen. Die Messungen sollen zur gleichen Zeit durchgeführt werden, vorzugsweise morgens nach dem Aufsehen, mittags und abends. Der Risikowert "gut", "gefährdet" und "hohes Risiko" kann wie in der nachfolgend wiedergegebenen Tabelle aus dem Venenfunktionsindex bestimmt werden, wobei ein Venenfunktoinsindex > 84 mit "gut" bewertet wird, ein Venenfunktionsindex von 68 bis 83 mit "ausreichend" und ein Venenfunktionsindex < 67 mit "mangelhaft":

| Risikowert | Anzahl der Venenfunktionsindexwerte in den jeweiligen Bereichen | | |
| --- | --- | --- | --- |
| | Venenfunktionsindex < 67 "mangelhaft" | Venenfunktionsindex von 68 - 83 "ausreichend" | Venenfunktionsindex > 85 "gut" |
| gut | 0-5 | 0 - 15 | 0-45 |
| gefährdet | 5 - 10 | 15 - 20 | |
| hohes Risiko | mehr als 10 | mehr als 20 | |

Der Risikowert wird also mit "hohes Risiko" bewertet, wenn mehr als 10 mal ein Venenfunktionsindex < 67 "mangelhaft" bestimmt worden ist oder wenn mehr als 20 mal ein Venenfunktionsindex von 68 bis 83 "ausreichend" bestimmt worden ist. Der Risikowert wird als "gefährdet" bestimmt, wenn 5 bis 10 mal ein Venenfunktionsindex < 67 "mangelhaft" bestimmt worden ist oder 15 bis 20 mal ein Venenfunktionsindex von 68 bis 83 "ausreichend". In den übrigen Fällen wird der Risikowert als "gut" bestimmt.

Ein Ausführungsbeispiel der Erfindung wird nachstehend anhand der beigefügten Zeichnung im einzelnen erläutert. In der Zeichnung zeigt:

Fig. 1    eine Vorrichtung zur Bestimmung der Lichtreflexion, insbesondere zur Bestimmung der Wiederauffüllzeit der Beinvenen, in einer schematischen Darstellung,

Fig. 2      einen Programmablaufplan für die Vorrichtung gemäß Fig. 1,

Fig. 3      den zeitlichen Verlauf der Lichtreflexion in einem typischen Fall,

Fig. 4      eine erste Ausführungsform eines Sensors in einem Querschnitt,

Fig. 5      die Platine für den Sensor gemäß Fig. 4 in einer Ansicht von unten,

Fig. 6      das Unterteil des Sensors gemäß Fig. 4 in einer Ansicht von unten,

Fig. 7      das Unterteil gemäß Fig. 6 in einer Ansicht von oben,

Fig. 8      das Oberteil des Sensors gemäß Fig. 4 - 7 in einer Ansicht von unten,

Fig. 9      das Oberteil gemäß Fig. 8 in einer Ansicht von oben,

Fig. 10    das Unterteil einer zweiten Ausführungsform des Sensors in einer Ansicht von unten,

Fig. 11    das Unterteil gemäß Fig. 10 in einer Ansicht von oben,

Fig. 12    das Oberteil für den Sensor gemäß Fig. 10 - 11 in einer Ansicht von unten und

Fig. 13    das Oberteil gemäß Fig. 12 in einer Ansicht von oben.

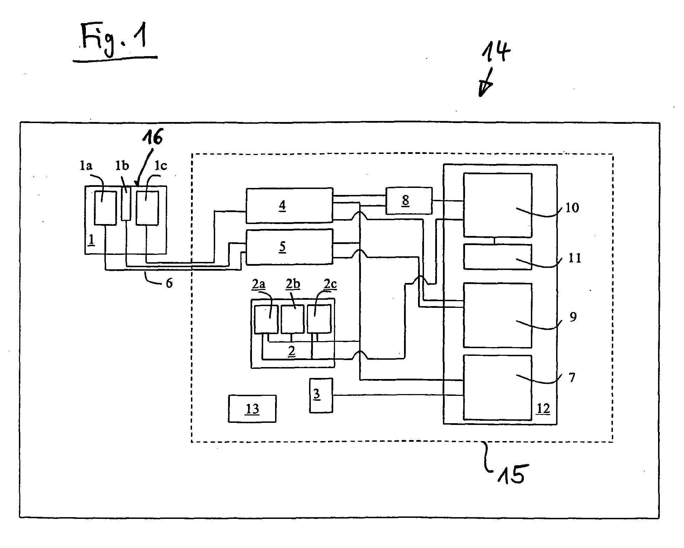

Die in Fig. 1 gezeigte Vorrichtung 14 zur Bestimmung der Lichtreflexion, insbesondere zur Bestimmung der Wiederauffüllzeit der Beivenen umfaßt ein Gerät 15 und einen Sensor 1, der durch abgeschirmte Leitungen 6 mit dem Gerät 15 verbunden ist. Der Sensor 1 umfaßt einen Sender 1a, nämlich eine NIR-LED in SMD-Technik, einen Empfänger 1c, nämlich eine großflächige Photodiode in SMD-Technik, und einen Kontaktstift 1b, der zum Potentialausgleich dient und durch den ein Ohmscher Kontakt zu der Fläche, mit der der Sensor in Kontakt bringbar ist, insbesondere zur Haut, herstellbar ist. Hierdurch kann die kapazitive Störeinstrahlung aufgrund des geringen Abstandes zwischen dem Sensor und der Fläche bzw. der Haut neutralisiert werden. Der Sender 1 a hat einen minimalen Abstand zu der der Fläche bzw. der Haut zugewandten Sensorfläche 16, so daß nur ein geringer Leistungsverlust entsteht. Der Empfänger 1c hat einen minimalen Abstand von der der Fläche bzw. der Haut zugewandten Sensor-Oberfläche 16; um eine größtmögliche Empfindlichkeit zu erreichen.

In dem Gerät 15 ist eine LED-Ansteuerung 5 vorgesehen, die über die abgeschirmten Leitungen 6 mit dem Sender 1a und dem Kontaktstift 1b verbunden ist und die dem Sender gepulste Ansteuersignale zuführt. Femer umfaßt das Gerät 15 einen Signalverstärker 4, der Empfangssignale von dem Empfänger 1c über eine abgeschirmte Leitung 6 empfängt.

In dem Gerät 15 ist eine Steuereinrichtung 12 vorhanden, die durch einen Mikrocontroller realisiert sein kann. Die Steuereinrichtung 12 umfaßt eine Ablaufsteuerung 7, insbesondere eine vollautomatische Ablaufsteuerung und Benutzerführung, eine Leistungsanpassung 9, insbesondere eine automatische Anpassung an die optischen Eigenschaften des Meßortes, eine Signalauswertung 10, insbesondere eine vollautomatische Auswertung und Bewertung der Rohmeßwertkurve, und eine Plausibilitätsprüfung 11, insbesondere eine automatische Erkennung von Meßoder Anwendungsfehlern, die mit der Signalauswertung 10 verbunden ist.

Der Signalverstärker 4 ist über einen Analog/Digital-Wandler 8 mit der Signalauswertung 10 verbunden. Er ist ferner mit der LED-Ansteuerung 5, einem Display 2, der Ablaufsteuerung 7 und der Leistungsanpassung 9 verbunden. Die LED-Ansteuerung ist mit dem Analog/Digital-Wandler 8, dem Display 2, der Ablaufsteuerung 7 und der Leistungsanpassung 9 verbunden.

Das Display 2 umfaßt eine numerische Anzeige 1 a, eine graphische Animation 2b und eine graphische Auswertung 2c. Die numerische Anzeige dient zur Anzeige der Ablaufsteuerung, der Benutzerführung, zur Ergebnisanzeige und zur Anzeige des Venenfunktionsindex. Die graphische Animation dient zur Anzeige der Ablaufsteuerung und zur Benutzerführung. Die graphische Auswertung dient zur Interpretation des Meßergebnisses. Numerische Anzeige 2a, graphische Animation 2b und graphische Auswertung 2c sind jeweils mit dem Signalverstärker 4, der LED-Ansteuerung 5, der Ablaufsteuerung 7 und dem Analog-/Digital-Wandler 8 verbunden. Die numerische Anzeige 2a und die graphische Auswertung 2c sind femer mit der Signalauswertung 10 verbunden.

Das Gerät 15 umfaßt ferner eine Taste 3, der mit der Ablaufsteuerung 7 verbunden ist. Es ist nur eine einzige Taste 3 vorhanden. In dem Gerät 15 ist also eine Einknopfbedienung realisiert.

Das Gerät 15 umfaßt ferner eine Stromversorgung 13, nämlich eine Batterie.

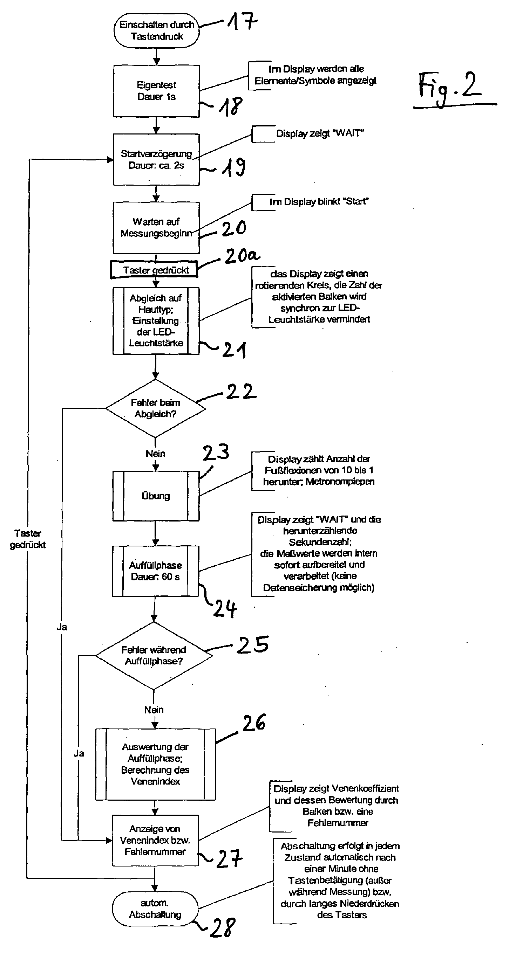

In Fig. 2 ist der Ablaufplan für die Vorrichtung gemäß Fig. 1 dargestellt. Der Vorgang beginnt im Block 17 mit dem Einschalten der Vorrichtung 14 durch einen Druck auf die Taste 3. Dadurch wird die Vorrichtung aktiviert. Während des anschließenden Eigentests 18 mit einer Dauer von 1 s werden im Display 2 alle Elemente/Symbole angezeigt. In der daran anschließenden Startverzögerung 19 mit einer Dauer von 2 s zeigt das Display 2 "WAIT".

Danach wartet das Gerät im Block 20 auf den Beginn der Messung. Im Display 2 blinkt während dieser Zeit die Aufforderung "Start".

Sobald die Bedienungsperson im Block 20a die Taste 3 drückt, um die Vorrichtung zu steuern, erfolgt im Block 21 der Abgleich auf den Hauttyp; die LED-Leuchtstärke wird eingestellt. Das Display 2 zeigt einen rotierenden Kreis, die Zahl der aktivierten Balken wird synchron zur LED-Leuchtstärke vermindert.

Nach der Durchführung des Abgleichvorgangs 21 wird im Block 22 abgefragt, ob beim Abgleich 21 ein Fehler aufgetreten ist. Wenn dies nicht der Fall ist, wird der Nein-Zweig durchlaufen, der zum Block 23 "Übung" führt. Die Person führt die Übung durch. Das Display 2 zählt die Anzahl der Fußflexionen von zehn bis eins herunter. Ein Tongeber kann in dieser Zeit ein Metronompiepen erzeugen.

In der anschließenden Auffüllphase 24 mit einer Dauer von 60 s zeigt das Display "WAIT" und eine von 60 herunterzählende Sekundenzahl. Die Meßwerte werden intern sofort aufbereitet und verarbeitet. Dabei kann eine Datenspeicherung durchgeführt werden. Dies ist allerdings nicht erforderlich.

Im anschließenden Block 25 wird abgefragt, ob während der Auffüllphase 24 ein Fehler aufgetreten ist. Wenn dies nicht der Fall ist, wird der Nein-Zweig durchlaufen, der zum Block 26 "Auswertung der Auffüllphase; Berechnung des Venenfunktionsindex" führt. Danach wird im Block 27 der Venenfunktionsindex angezeigt. Wenn in der Abfrage 25 ein Fehler während der Auffüllphase erkannt worden ist, wird der Ja-Zweig durchlaufen, der unter Umgehung des Blocks 26 unmittelbar zum Block 27 führt. In diesem Fall wird eine Fehlernummer angezeigt.

Wenn im Block 22 ein Fehler beim Abgleich 21 festgestellt wurde, wird der Ja-Zweig durchlaufen, der vom Block 22 unmittelbar zum Block 27 führt. Auch in diesem Fall wird im Block 27 eine Fehlernummer angezeigt.

Im Block 27 zeigt das Display 2 den Venenfunktionsindex und dessen Bewertung durch Balken bzw. eine Fehlernummer.

Die Anzeige des Blocks 27 bleibt während einer vorgegebenen Zeit von beispielsweise einer Minute erhalten. Wenn während dieser Zeit die Taste 3 betätigt wird, springt das Programm zurück zum Block 19 "Startverzögerung". Andernfalls, wenn also während der vorgegebenen Zeit von beispielsweise einer Minute die Taste 3 nicht erneut betätigt worden ist, wird im Block 28 eine automatische Abschaltung des Geräts durchgeführt. Zusätzlich kann vorgesehen sein, daß durch ein langes Niederdrücken der Taste 3 während eines vorgegebenen Zeitraums von beispielsweise drei Sekunden die automatische Abschaltung 28 sofort durchgeführt wird.

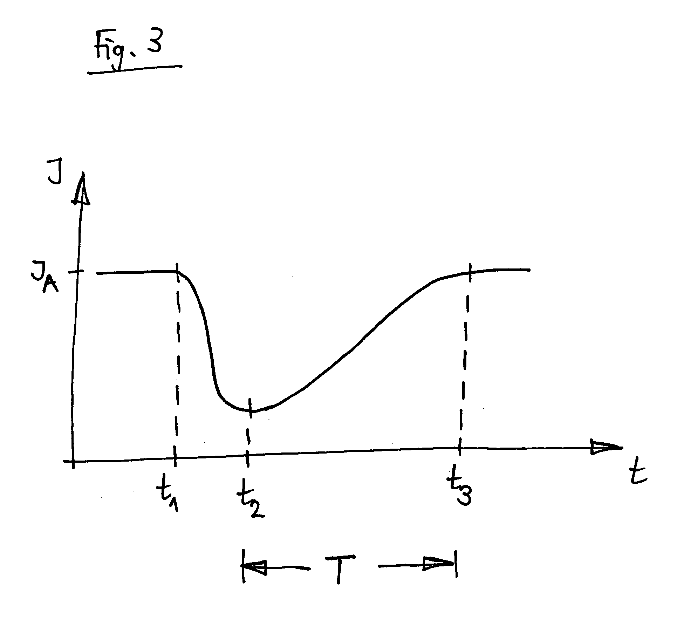

Fig. 3 zeigt einen zeitlichen Verlauf der vom Empfänger 1c gemessenen Intensität I. Die Intensität I hat zunächst den Anfangswert $I_A$. Nach Beginn der Übung im Zeitpunkt $t_1$ sinkt der Intensitätswert I ab. Er liegt am Ende der Übung zum Zeitpunkt $t_2$ unter dem Anfangswert $I_A$. Der Zeitpunkt $t_2$ ist gleichzeitig der Beginn der Auffüllphase 24, also der Ausgangszeitpunkt. Der Intensitätswert I steigt anschließend an, bis er zum Zeitpunkt $t_3$ den Anfangswert $I_A$ wieder erreicht oder zumindest annähernd wieder erreicht. Der Zeitpunkt $t_3$ markiert den Endzeitpunkt. Zwischen den Zeitpunkten $t_2$ und $t_3$ verstreicht der Zeitraum T, der durch die Signalauswerteeinrichtung 10 bestimmt wird.

Der Zeitpunkt $t_3$ und damit der Zeitraum T kann unter Heranziehung der Anfangsintensität $I_A$ bestimmt werden. Es ist allerdings auch möglich, den Zeitpunkt $t_3$ und damit den Zeitraum T ausschließlich aus der Kurvenform, also der Form der Intensitäts-Zeit-Kurve gemäß Fig. 3 zu bestimmen.

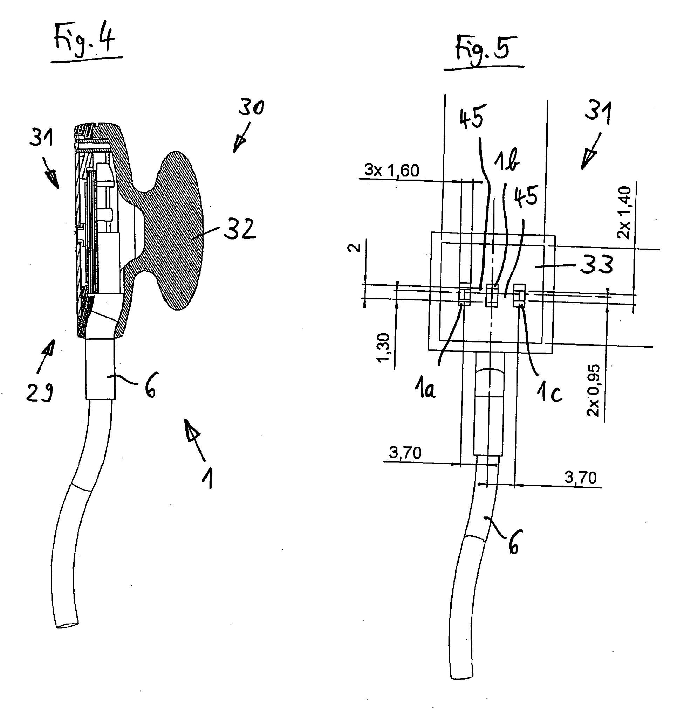

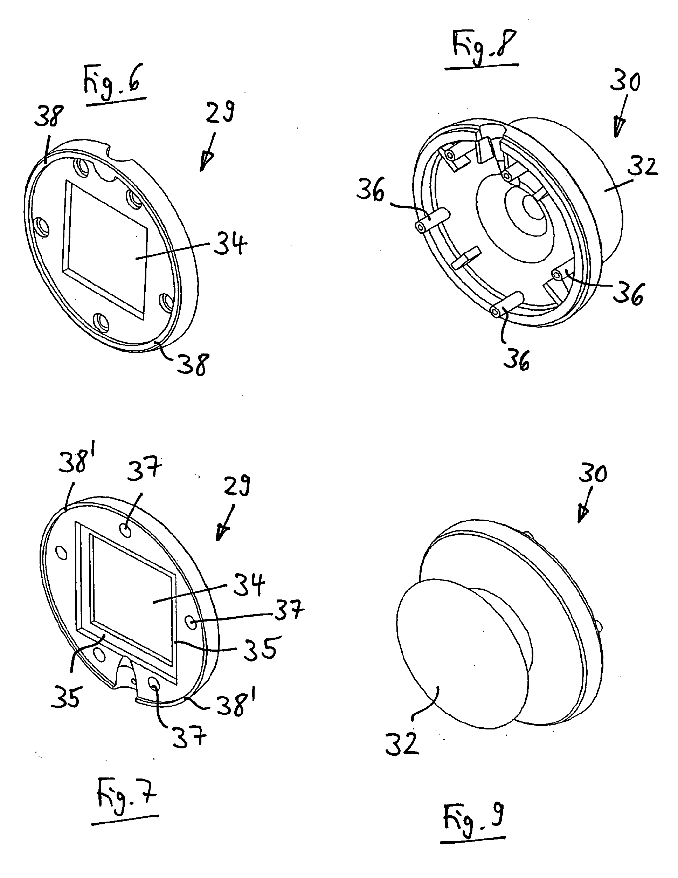

Die Figuren 4 bis 9 zeigen eine erste Ausführungsform eines Sensors 1. Der Sensor 1 besteht aus einem Unterteil 29, einem Oberteil 30 und einer Platine 31. Das Unterteil 29 und das Oberteil 30, die miteinander verbunden sind, nehmen zwischen sich die Platine 31 auf. Das Oberteil 30 umfaßt ein Griffstück 32. Die Platine 31 umfaßt einen Chip 33, der mit der abgeschirmten Leitung 6 verbunden ist.

Auf dem Chip 33 sind ein Sender 1 a, ein Empfänger 1c und ein Kontaktstift 1 b vorgesehen. Das Unterteil 29 besitzt eine quadratische Aussparung 34 mit einem umlaufenden Vertiefung 35, in welcher der Chip 33 zu liegen kommt. Am Oberteil 30 sind Hohlstifte 36 angeformt, die in entsprechenden Löchern 37 im Unterteil 29 zu liegen kommen und die dort festgeschraubt werden können. Auf der Unterseite des Unterteils 29 ist ein erhöhter umlaufender Rand 38 vorgesehen, der mit der zu messenden Oberfläche bzw. der Haut in Kontakt gebracht werden kann. An der Oberseite des Unterteils befindet sich ein umlaufender Rand 38'.

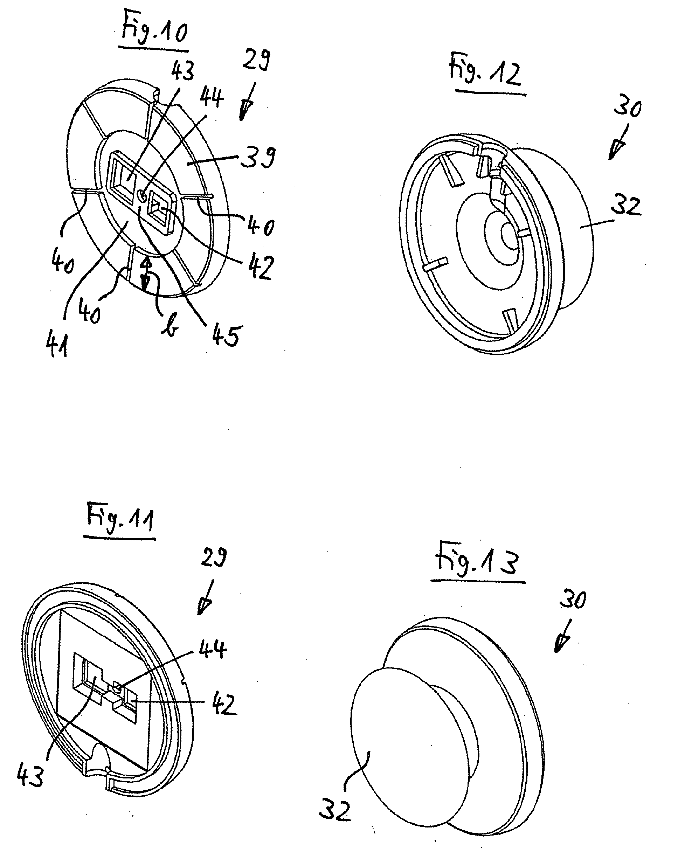

In den Figuren 10 bis 13 ist eine abgewandelte Ausführungsform gezeigt, bei der entsprechende Teile mit entsprechenden Bezugszeichen versehen sind. Das Unterteil 29 weist auf seiner Unterseite eine ringförmige Kontaktfläche 39 auf, deren Breite b etwa 2/5 des Radius des Unterteils 29 beträgt und die durch sechs Kerben 40 unterbrochen ist. Wenn das Unterteil 29 auf der zu messenden Oberfläche bzw.

Hautoberfläche anliegt, bilden die Kerben 40 Luftkanäle von der Außenseite zum Inneren 41 des Unterteils 29, in dem eine Aussparung 42 für den Sender 1a, eine Aussparung 43 für den Empfänger 1c und eine dazwischen liegende Aussparung 44 für den Kontaktstift 1b vorgesehen sind. Durch den aus Messing bestehenden Kontaktstift 1b wird ein elektrischer Kontakt zu der zu messenden Oberfläche bzw. Hautoberfläche hergestellt. Gleichzeitig wird durch diesen Kontaktstift 1b die Platine 31 am Unterteil 29 und Oberteil 30 fixiert. Auf der ringförmigen Kontaktfläche 39 kann ein Klebering aufgebracht werden, der auf beiden Seiten eine klebende Oberfläche hat, so daß er sowohl auf der Ringfläche 39 als auch auf der zu messenden Oberfläche bzw. Hautoberfläche fixiert wird und auf diese Weise den Sensor zuverlässig auf der Oberfläche festhält.

Zwischen dem Sender 1a und dem Empfänger 1c ist ein Steg 45 vorgesehen, der eine optische Trennung bewirkt. Der Sensor kann oberhalb des Fußknöchels in einem Abstand von vorzugsweise etwa 8 cm positioniert werden.

**Patentansprüche**

1. Vorrichtung zur Bestimmung der Lichtreflexion, insbesondere zur Bestimmung der Wiederauffüllzeit der Beinvenen,
   **gekennzeichnet durch**
   einen Sensor (1) zum Erfassen der Lichtreflexion zu verschiedenen Zeitpunkten
   und eine Signalauswerteeinrichtung (10) zum Bestimmen des Zeitraums (T) zwischen einem Ausgangszeitpunkt ($t_2$) und einem Endzeitpunkt ($t_3$).

2. Vorrichtung nach Anspruch 1, **dadurch gekennzeichnet, daß** die Signalauswerteeinrichtung (10) als Endzeitpunkt ($t_3$) den Zeitpunkt bestimmt, zu dem die Lichtreflexion (I) einen Anfangswert ($I_A$), den sie zu einem Anfangszeitpunkt ($t_1$) aufweist, wieder erreicht oder annähernd wieder erreicht.

3. Vorrichtung nach Anspruch 1 oder 2, **dadurch gekennzeichnet, daß** die Signalauswerteeinrichtung (10) den Endzeitpunkt ($t_3$) durch eine Auswertung von zu verschiedenen Zeitpunkten erfaßten Lichtreflexionswerten (I) bestimmt.

4. Vorrichtung nach einem der vorhergehenden Ansprüche **gekennzeichnet durch** eine Signalauswerteeinrichtung (10) zum Bestimmen eines Venenfunktionsindex (V) aus dem bestimmten Zeitraum (T).

5. Vorrichtung nach Anspruch 4, **dadurch gekennzeichnet, daß** für einen bestimmten Zeitraum (T) von bis zu einer Mindestzeit, vorzugsweise bis zu 12 s, vorzugsweise 9 s bis 11 s, vorzugsweise 10 s, der Venenfunktionsindex (V) einen Mindestwert beträgt.

6. Vorrichtung nach Anspruch 4 oder 5, **dadurch gekennzeichnet, daß** für einen bestimmten Zeitraum (T) von über einer Höchstzeit, vorzugsweise über 30 s, vorzugsweise 32 s bis 37 s, vorzugsweise 34,5 s der Venenfunktionsindex (V) einen Höchstwert beträgt.

7. Vorrichtung nach einem der Ansprüche 4 bis 6, **dadurch gekennzeichnet, daß** für bestimmte Zeiträume (T) zwischen der Mindestzeit und der Höchstzeit der Venenfunktionsindex (V) einen Zwischenwert beträgt.

8. Vorrichtung nach einem der Ansprüche 4 bis 7, **dadurch gekennzeichnet, daß** der Mindestwert 10 und Höchstwert 100 beträgt.

9. Vorrichtung nach einem der Ansprüche 4 bis 8, **dadurch gekennzeichnet, daß** die Signalauswerteeinrichtung (10) den Venenfunktionsindex (V) als Funktion des ermittelten Zeitraums (T) bestimmt (V = V (T)), vorzugsweise nach der Formel

$$V = a \cdot (T-b).$$

10. Vorrichtung nach einem der vorhergehenden Ansprüche, **gekennzeichnet durch** eine Signalauswerteeinrichtung (10) zum Bestimmen eines Anzeigewerts aus dem bestimmten Zeitraum (T) oder aus dem Venenfunktionsindex (V) und eine Anzeigeeinrichtung (2) zum Anzeigen dieses Anzeigewertes.

11. Vorrichtung nach Anspruch 10, **dadurch gekennzeichnet, daß** der Anzeigewert mehrere Bewertungsgrößen umfaßt, vorzugsweise drei Bewertungsgrößen, vorzugsweise die Bewertungsgrößen "gut", "ausreichend" und "mangelhaft".

**12.** Vorrichtung nach Anspruch 11, **dadurch gekennzeichnet, daß** der Anzeigewert den Wert "mangelhaft" für bestimmte Zeiträume (T) bis zu 25 s, "ausreichend" für bestimmte Zeiträume (T) von 25 s bis 31,5 s und "gut" für bestimmte Zeiträume (T) von über 31,5 s aufweist.

**13.** Vorrichtung nach einem der vorhergehenden Ansprüche, **gekennzeichnet durch** ein Betätigungselement (3) zum Aktivieren (17) und/oder Steuern (20a) der Vorrichtung.

**14.** Vorrichtung nach Anspruch 13, **gekennzeichnet durch** ein einziges Betätigungselement (3) zum Aktivieren (17) und Steuern (20a) der Vorrichtung:

**15.** Vorrichtung nach einem der vorhergehenden Ansprüche, **dadurch gekennzeichnet, daß** der Sensor (1) einen Sender (1a) und einen Empfänger (1c) umfaßt, vorzugsweise nur einen Sender (1a) und nur einen Empfänger (1c).

**16.** Vorrichtung nach Anspruch 15, **dadurch gekennzeichnet, daß** der Sensor (1) einen Potentialkontakt, vorzugsweise einen Kontaktstift (1b) umfaßt.

**17.** Vorrichtung nach Anspruch 15 oder 16, **dadurch gekennzeichnet, daß** der Sensor (1) eine vorzugsweise ringförmige Kontaktfläche (38, 39) umfaßt.

**18.** Vorrichtung nach einem der Ansprüche 15 bis 17, **dadurch gekennzeichnet, daß** der Sensor (1) und/oder die zu untersuchende Oberfläche bzw. Haut gekühlt ist.

**19.** Vorrichtung nach einem der Ansprüche 15 bis 18, **dadurch gekennzeichnet, daß** der Sensor (1) belüftbar ist.

**20.** Vorrichtung nach einem der Ansprüche 15 bis 19, **dadurch gekennzeichnet, daß** in und/oder an dem Sensor (1) Belüftungskanäle vorgesehen sind.

**21.** Vorrichtung nach einem der Ansprüche 15 bis 20, **dadurch gekennzeichnet, daß** in der vorzugsweise ringförmigen Kontaktfläche (38, 39) Kerben (40) vorgesehen sind.

**22.** Vorrichtung nach einem der Ansprüch 15 bis 21, **dadurch gekennzeichnet, daß** der Sensor (1), insbesondere die vorzugsweise ringförmige Kontaktfläche (38, 39) des Sensors (1), ein wärmeleitendes Material umfaßt.

**23.** Verfahren zum Bestimmen eines Risikowerts, insbesondere eines Risikowerts für die Venenfunktion einer Person, **dadurch gekennzeichnet,**
**daß** die Wiederauffüllzeit der Beinvenen einer Person zu verschiedenen Zeiten bestimmt wird, daß die bestimmten Wiederauffüllzeiten und/oder aus der jeweiligen Wiederauffüllzeit bestimmte Venenfunktionsindexwerte gespeichert werden und daß aus den Wiederauffüllzeiten bzw. Venenfunktionsindexwerten ein Risikowert bestimmt wird.

**24.** Verfahren nach Anspruch 23, **dadurch gekennzeichnet, daß** die Zeiten, zu denen die Wiederauffüllzeiten bestimmt werden, gespeichert werden.

**25.** Vorrichtung nach Anspruch 23 oder 24, **dadurch gekennzeichnet, daß** der Risikowert durch ein vorzugsweise statistisches Auswerteverfahren bestimmt wird.

**26.** Vorrichtung nach einem der Ansprüche 23 bis 25, **dadurch gekennzeichnet, daß** der Risikowert mehrere Bewertungsgrößen umfaßt, vorzugsweise drei Bewertungsgrößen, vorzugsweise die Bewertungsgrößen "gut", "gefährdet" und "hohes Risiko".

**27.** Vorrichtung zum Bestimmen eines Risikowerts, insbesondere eines Risikowerts für die Venenfunktion einer Person,
**gekennzeichnet durch**
eine Vorrichtung zum Bestimmen der Wiederauffüllzeit, insbesondere der Wiederauffüllzeit der Beinvenen, insbesondere eine Vorrichtung nach einem der Ansprüche 1 bis 22,
einen Speicher zum Speichern der zu verschiedenen Zeiten bestimmten Wiederauffüllzeiten und/oder zum Speichern eines aus der jeweiligen Wiederauffüllzeit bestimmten Venenfunktionsindex
und eine Risikowert-Bestimmungseinrichtung zum Bestimmen des Risikowerts aus den Wiederauffüllzeiten bzw. den Venenfunktionsindexwerten.

**28.** Vorrichtung nach Anspruch 27, **dadurch gekennzeichnet, daß** die Zeiten, zu denen die Wiederauffüllzeiten bestimmt werden, in dem Speicher gespeichert werden.

**29.** Vorrichtung nach Anspruch 27 oder 28, **dadurch gekennzeichnet, daß** der Risikowert mehrere Bewertungsgrößen umfaßt, vorzugsweise drei Bewertungsgrößen, vorzugsweise die Bewertungsgrößen "gut", "gefährdet" und "hohes Risiko".

Fig. 1

**Fig. 2**

Flussdiagramm:

- Einschalten durch Tastendruck — 17
- Eigentest Dauer 1s — 18 → Im Display werden alle Elemente/Symbole angezeigt
- Startverzögerung Dauer: ca. 2s — 19 → Display zeigt "WAIT"
- Warten auf Messungsbeginn — 20 → Im Display blinkt "Start"
- Taster gedrückt — 20a
- Abgleich auf Hauttyp; Einstellung der LED-Leuchtstärke — 21 → das Display zeigt einen rotierenden Kreis, die Zahl der aktivierten Balken wird synchron zur LED-Leuchtstärke vermindert
- Fehler beim Abgleich? — 22
  - Nein ↓
  - Ja → (Taster gedrückt)
- Übung — 23 → Display zählt Anzahl der Fußflexionen von 10 bis 1 herunter; Metronompiepen
- Auffüllphase Dauer: 60 s — 24 → Display zeigt "WAIT" und die herunterzählende Sekundenzahl; die Meßwerte werden intern sofort aufbereitet und verarbeitet (keine Datenseicherung möglich)
- Fehler während Auffüllphase? — 25
  - Nein ↓
  - Ja →
- Auswertung der Auffüllphase; Berechnung des Venenindex — 26
- Anzeige von Venenindex bzw. Fehlernummer — 27 → Display zeigt Venenkoeffizient und dessen Bewertung durch Balken bzw. eine Fehlernummer
- autom. Abschaltung — 28 → Abschaltung erfolgt in jedem Zustand automatisch nach einer Minute ohne Tastenbetätigung (außer während Messung) bzw. durch langes Niederdrücken des Tasters

Fig. 3

Fig.4

Fig.5

31

30

32

29

6

1

3x 1,60

45

1b

45

31

2x 1,40

2

33

1,30

1a

1c

2x 0,95

3,70

3,70

6

Fig.6

38

29

34

38

Fig.8

30

36

32

36

36

38'   37

29

34

37

35

35   38'

37

Fig.7

30

32

Fig.9

Fig. 10

Fig. 11

Fig. 12

Fig. 13

# EP 1 442 702 A1

**Europäisches Patentamt**

## EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 04 00 2376

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X<br>Y | EP 0 359 972 A (NATTERMANN A & CIE)<br>28. März 1990 (1990-03-28)<br><br>* Spalte 2, Zeile 40-54 *<br>* Spalte 3, Zeile 26 - Spalte 4, Zeile 25 *<br>* Abbildungen 1,2,4,5 *<br>--- | 1-3,10,<br>14,15<br>16-22 | A61B5/0295 |
| X | DE 36 09 075 A (SCHMITT HANS J PROF DR RER NAT) 24. September 1987 (1987-09-24)<br><br>* Spalte 3, Zeile 63 - Spalte 5, Zeile 2 *<br>* Abbildungen 1,3 *<br>--- | 1-4,10,<br>11,<br>13-15,<br>27-29 | |
| Y | US 5 697 367 A (LEWIS GARY D ET AL)<br>16. Dezember 1997 (1997-12-16)<br>* Spalte 2, Zeile 66 - Spalte 3, Zeile 9 *<br>* Spalte 4, Zeile 36-65 *<br>--- | 16 | |
| Y | EP 0 337 317 A (NATTERMANN A & CIE)<br>18. Oktober 1989 (1989-10-18)<br>* Spalte 4, Zeile 17-41 *<br>* Abbildungen 1,2 *<br>--- | 17-22 | RECHERCHIERTE SACHGEBIETE (Int.Cl.7)<br><br>A61B |
| | -/-- | | |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

1-22,27-29

Unvollständig recherchierte Patentansprüche:

Nicht recherchierte Patentansprüche:

23-26

Grund für die Beschränkung der Recherche:

Artikel 52 (4) EPÜ - Diagnostizierverfahren, die am menschlichen oder tierischen Körper vorgenommen werden

| Recherchenort | Abschlußdatum der Recherche | Prüfer |
|---|---|---|
| BERLIN | 29. April 2004 | Völlinger, M |

**Europäisches Patentamt**

## EUROPÄISCHER TEILRECHERCHENBERICHT

Nummer der Anmeldung

EP 04 00 2376

| | EINSCHLÄGIGE DOKUMENTE | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| A | DE 31 00 610 A (BLAZEK VLADIMIR;WIENERT VOLKER) 29. Juli 1982 (1982-07-29) * Seite 9, letzter Absatz - Seite 10, Absatz 1 * ----- | 1 | |
| | | | **RECHERCHIERTE SACHGEBIETE** (Int.Cl.7) |

EPO FORM 1503 03.82 (P04C12)

## ANHANG ZUM EUROPÄISCHEN RECHERCHENBERICHT
## ÜBER DIE EUROPÄISCHE PATENTANMELDUNG NR.

EP 04 00 2376

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-04-2004

| Im Recherchenbericht angeführtes Patentdokument | | Datum der Veröffentlichung | Mitglied(er) der Patentfamilie | | Datum der Veröffentlichung |
|---|---|---|---|---|---|
| EP 0359972 | A | 28-03-1990 | DE | 3827501 A1 | 12-04-1990 |
| | | | AT | 113811 T | 15-11-1994 |
| | | | DE | 58908625 D1 | 15-12-1994 |
| | | | EP | 0359972 A1 | 28-03-1990 |
| DE 3609075 | A | 24-09-1987 | DE | 3609075 A1 | 24-09-1987 |
| | | | AT | 111326 T | 15-09-1994 |
| | | | CA | 1312479 C | 12-01-1993 |
| | | | DE | 3750517 D1 | 20-10-1994 |
| | | | DK | 140887 A | 19-09-1987 |
| | | | EP | 0238065 A2 | 23-09-1987 |
| | | | US | 5009231 A | 23-04-1991 |
| US 5697367 | A | 16-12-1997 | WO | 9611626 A1 | 25-04-1996 |
| | | | US | 5795292 A | 18-08-1998 |
| EP 0337317 | A | 18-10-1989 | DE | 3811689 C1 | 01-06-1989 |
| | | | DK | 138089 A | 08-10-1989 |
| | | | EP | 0337317 A1 | 18-10-1989 |
| DE 3100610 | A | 29-07-1982 | DE | 3100610 A1 | 29-07-1982 |
| | | | AT | 15320 T | 15-09-1985 |
| | | | DE | 3172170 D1 | 10-10-1985 |
| | | | EP | 0063649 A1 | 03-11-1982 |
| | | | JP | 58036529 A | 03-03-1983 |
| | | | US | 4494550 A | 22-01-1985 |

EPO FORM P0461

Für nähere Einzelheiten zu diesem Anhang : siehe Amtsblatt des Europäischen Patentamts, Nr.12/82